# EUROPEAN PATENT SPECIFICATION

(11) **EP 0 900 048 B1**
(45) Date of publication and mention of the grant of the patent: **24.08.2005**
(21) Application number: 98911410.3
(22) Date of filing: 25.02.1998
(51) Int. Cl.: A61B 5/06, A61B 5/113

(54) **IMAGE-GUIDED THORACIC THERAPY APPARATUS**
VORRICHTUNG ZUR BILDUNTERSTÜTZTEN THORAX-THERAPIE
DISPOSITIF DE THERAPIE DU THORAX GUIDEE PAR IMAGE

(30) Priority: 25.02.1997 US 38497 P
(43) Date of publication of application: 10.03.1999
(73) Proprietor: Biosense Webster, Inc., California 91765 (US)
(72) Inventor: ACKER, David, E., Setauket, NY 11733 (US)
(74) Representative: Mercer, Christopher Paul
(86) International application number: PCT/US1998/003660
(87) International publication number: WO 1998/036684

(56) References cited:
- WO-A-90/13259
- WO-A-96/41119
- DE-U- 9 212 636
- US-A- 4 180 059
- US-A- 4 344 436
- US-A- 4 989 608
- US-A- 5 271 055
- US-A- 5 474 075
- US-A- 5 482 042
- US-A- 5 577 502

## Description

### TECHNICAL FIELD

The present invention relates to an apparatus for performing medical procedures in the thorax of a medical or veterinary patient.

### BACKGROUND ART

Some common medical procedures require the ability to operate on a specific location in the thorax, including locations in the respiratory system, such as the lungs, bronchi and immediately surrounding tissues. For example, needle aspiration biopsies have been performed heretofore using an endoscope inserted through the trachea into a bronchus. The needle is advanced through the endoscope through the bronchial wall to sample tissue in a lymph node within the lung parenchyma near the exterior surface of the bronchus. The physician can monitor placement of the endoscope and the biopsy needle using the optical system of the endoscope. As the endoscope is advanced toward the area to be sampled, the physician can determine where the tip of the endoscope lies by observing features of the airway itself. However, it is difficult to place a biopsy needle within a particular lymph node using this approach. The physician cannot see the lymph nodes, which lie outside of the airway. Therefore, the physician can only position the endoscope tip and the biopsy needle at an approximate position, near the location of the lymph node to be biopsied. For this reason, there has been a significant need for improvement in the reliability of needle aspiration biopsies of the lymph nodes surrounding the respiratory tract. There have been similar needs for improvement in other biopsies procedures using a probe advanced into the body, such as a biopsy needle or biopsy forceps to sample tissues in the vicinity of the respiratory tract. There have been similar needs for improvement in other procedures where a probe is advanced into the tissues of the thorax for other purposes as, for example, to perform surgical procedures on these tissues or to administer drugs within these tissues.

Some procedures heretofore have used imaging during advancement of the probe to provide guidance. Thus, as the probe is advanced, the probe and the body are imaged using conventional imaging techniques such as fluoroscopy or magnetic resonance imaging. This allows the physician to observe the relationship between the position of the probe and the surrounding tissues. These procedures have the disadvantage that the imaging apparatus is occupied for the entire time required to perform the procedure. Moreover, the use of fluoroscopic or other x-ray based imaging modalities during the procedure exposes the physician and the patient to radiation.

As described, for example, in U.S. Patents 5,558,091, 5,391,199; 5,443,489; and in PCT International Publication WO 96/05768, the position, orientation or both of the distal end of a probe can be determined by using one or more field transducers such as a Hall effect or magnetoresistive device, coil or other antenna carried on the probe, typically at or adjacent the distal end of the probe. One or more additional field transducers are disposed outside the body in an external frame of reference. The field transducers preferably are arranged to detect or transmit non-ionizing fields or field components such as a magnetic field, electromagnetic radiation or acoustical energy such as ultrasonic vibration. By transmitting the field between the external field transducers and the field transducers on the probe, characteristics of field transmission between these devices can be determined. The position and/or orientation of the sensor in the external frame of reference can then be deduced from these transmission characteristics. Because the field transducer of the probe allows determination of the position of the probe, such transducer is also referred to as a "position sensor".

As described, for example, in the aforementioned U.S. Patent 5,558,091, the frame of reference of the external field transducers can be registered with the frame of reference of imaging data such as magnetic resonance imaging data, computerized axial tomographic data, or conventional x-ray image data. The probe position and orientation data derived by field transmission can be displayed as a representation of the probe superimposed on an image of the patient's body. The physician can use this information to guide the probe to the desired location within the patient's body, and to monitor its orientation during treatment or measurement of the body structure. This arrangement greatly enhances the ability of the physician to navigate the distal end of the probe through bodily structures. Because it does not require acquisition of an optical image of the surrounding tissues for navigation purposes, it can be used with probes which are too small to accommodate optical elements, and can be used for navigation of the probe within solid or semisolid tissues. The transducer-based system also avoids the difficulties associated with navigation of a probe by continuous imaging of the probe and patient during the procedure. For example, it avoids exposure to ionizing radiation inherent in fluoroscopic systems.

Some additional problems are encountered in use of systems of this type for procedures in the thorax near the respiratory system. As the patient breathes, the positions, sizes and shapes of the thoracic organs change. Thus, if an image of the patient is acquired at one stage of the respiratory cycle, the image data does not accurately represent the patient during other stages. Therefore, if the position of the probe is detected while the patient is in one stage of the respiratory cycle, and this probe position data is combined with patient image data from another stage of the respiratory cycle to provide an image with a representation of the probe superposed thereon, the location of the probe relative to the surrounding organs will be depicted inaccurately. As described in International Publication WO 97/29709, problems of this nature can be avoided by positioning a first probe, referred to as a "site probe" within the body of the patient at a location to be treated, and providing a further probe, referred to as an "instrument probe" for performing the medical procedure. The site probe is positioned within the body at the location to be treated as, for example, at a location to be biopsied. Using a location system such as the magnetic location systems discussed in the aforementioned patents, the locations of both probes are monitored during the medical procedure. Therefore, the distance and direction from the instrument probe to the site probe are known during the medical procedure, despite any motion caused by the patient's breathing. Using that directional and distance information, the physician can navigate the instrument probe to the site probe.

PCT Publication WO 97/29682 refers to systems for determining the "physiological motion" such as breathing motion or cardiac motion of a portion of the body in which a probe is situated. Using a device such as a belly strap to sense breathing motion, the system selects a "correct" image from a set of previously obtained images at each instant during the procedure, or interpolates between images. Thus, the displayed image always reflects the actual size and shape of the organs at the instant in question. Accordingly, the representation of the probe can be accurately superposed on the display image.

U.S. Patent 5,577,502 discloses a system in which the position of the subject's chest is monitored by devices such as optical, ultrasound or mechanical tracking elements. Based on that positional tracking, the image used in a superposition system is distorted so as to provide a corrected image which changes as the subject breathes. The position of the probe can be superposed on the corrected image. Systems of this type require considerable computation to distort the reference image as the patient moves through various stages of the respiratory cycle. Moreover, additional equipment is required for tracking the position of the patient's chest. In an alternative approach also discussed in the '502 patent, a series of images is acquired at numerous stages of the respiratory cycle. As the patient moves through different stages of the respiratory cycle, different images are employed. This approach multiplies the task of acquiring and storing the image data. Moreover, this approach can only be used if a set of multiple images exists. For example, where the patient is subjected to a conventional diagnostic imaging procedure such as an MRI or CT imaging, a single set of image data representing the patient at only one stage of the respiratory cycle generally is acquired. The need for a biopsy or other procedure using a probe advanced into the patient may only be apparent after that image has been evaluated. To acquire a series of images, the patient must be subjected to further imaging procedures before the interventional procedure using the probe can begin.

In US 5,482,042, there is disclosed a medical imaging apparatus including a respiration depth detecting device for detecting a respiration depth of a patient and an indication device for indicating the detected respiration depth. The respiration depth detection device includes an ultrasonic measuring device for measuring a position of a surface of the body of the patient, a device for detecting the maximum and minimum extreme values of position, and a device for calculating the respiration depth based on the maximum and minimum values.

Thus, despite these and other efforts in the art, further improvements in interventional procedures and apparatus for performing the same would be desirable.

### DISCLOSURE OF THE INVENTION

According to the present invention, there is provided apparatus according to claim 1.

Thus, apparatus of the present invention can utilise the same position measuring devices as employed in determining the probe position to determine the respiratory cycle.

Typically, the means for detecting is arranged to detect when the patient is in the selected state by determining whether the position of the reference point matches the extreme position within a preselected tolerance. For example, the selected stage of the respiratory cycle may be the minimum inspiration state, i.e. the state achieved at the end of exhalation during a normal breathing cycle. In this case, the system may select the position of the reference point at which the patient's front chest wall is closest to the patient's back. If the patient is lying in a supine position, with his or her back on a table, the system may select the position where a reference point on the patient's front chest wall is closest to the table.

The probe can be advanced within an airway as, for example, to pass a part of the probe through the wall of the airway to treat tissue at a target location within the tissues surrounding the airway. An example of such a target location is the lymph nodes outside of the airway. The probe may include an endoscope and a needle. The endoscope can be advanced until the endoscope is positioned at the wall of the airway adjacent the target location and then to advance the needle through the wall of the airway.

The image may include a plurality of identifiable points on the body of the patient which have positions substantially unaffected by the respiratory cycle of the patient. The identifiable points may, for example, include a point on the scapula or on the sternum.

Typically, the computer is adapted to transform the disposition of the probe into the image frame of reference. Desirably, the computer may be arranged to display a perspective image of the airway and surrounding tissues so that the position of the probe and the trajectory for moving the probe to engage the target location can be visualized by viewing the displayed image.

Because the disposition of the probe used as the basis for the superposed representation is acquired at the same stage in the respiratory cycle as the image, the motion artefact or inaccuracy caused by motion due to the respiratory cycle is eliminated. The apparatus according to the invention thus provides a solution to the motion artefact problem which does not require acquisition of multiple images for massive manipulation of image data to distort an image. The system is compatible with standard images acquired for diagnostic purposes which represent only one stage in the respiratory cycle.

When in use, as the probe of the present invention is advanced the patient may be instructed to hold his or her breath at the prescribed respiratory state. Thus, while the patient holds the prescribed point in the respiratory state, the system will continually acquire new positions of the probe and will continually update the superposed representation of the probe on the image. If the patient momentarily deviates from the prescribed stage of the respiratory cycle the system will stop generating new superposed positions of the probe representation on the image and preferably will provide a warning to the physician.

### MODES FOR CARRYING OUT THE INVENTION

Apparatus according to one embodiment of the present invention includes a probe. The probe may incorporate essentially any device which can be inserted or advanced into the body to perform a medical procedure, such as treatment, measurement or observation. As used herein, the term "treatment" includes capturing samples of tissues or materials present within the body, and thus includes biopsies. The probe desirably includes a conventional endoscope having a tubular body. Body has a handle portion affixed to a proximal end of the body and has a distal portion remote from handle. Body has a bore extending longitudinally from its proximal end to its distal end and open to the outside through handle. Body may incorporate a flexible section adjacent the distal end, so that the distal end can be bent or pivoted relative to the remainder of the body. The endoscope may incorporate devices for bending the distal end of the body so as to steer the device as it is advanced into the patient's anatomy. The endoscope may further include a fiber optic or television system for visually observing the anatomical features of the patient at the distal end of the endoscope.

The probe further includes a conventional intrabody medical tool such as a biopsy needle or other surgical tool operable from the proximal end or handle of the device. Merely by way of example, instead of a biopsy needle, the tool may be any conventional surgical tool of the type commonly used in endoscopic, arthroscopic, a laparoscopic surgical procedures; a biopsy forceps or other sampling device; a needle, catheter or other drug delivery device; a measuring instrument such as a thermometer or electrical potential measuring electrode; a device for applying therapeutic radiation; or any other device which can be used to treat, measure or observe structures within the body of a living subject. Needle is arranged so that it can be advanced to an operative position outside of the distal end of body. Needle is arranged so that it can be manipulated and controlled from the proximal end or handle of the body. Thus, the needle is connected to a manipulating handle by conventional control elements or linkages. Other expedients for manipulating and controlling a tool at the distal end of body can be employed as, for example, electrical, electronic or optical control linkages. Alternatively, a tool can be mounted in fixed position on body or formed integrally therewith as, for example, where body is equipped with a cutting blade.

A probe field transducer or position sensor is mounted in probe body adjacent the distal end thereof. Transducer may be a sensor arranged to detect magnetic or electromagnetic fields. For example, the sensor may be a multiaxis, solid-state position sensor of the type disclosed in the aforementioned U.S. Patent 5,558,091. Such a sensor incorporates a plurality of transducers sensitive to magnetic field components in mutually orthogonal directions. Other suitable position sensors include coils as disclosed in the aforementioned U.S. Patent 5,391,199 and in PCT Application PCT/US95/01103, now published as PCT International Publication WO 96/05768,.

Such coils may be provided as a single coil or as a plurality of orthogonal coils capable of detecting field components in orthogonal directions. Position sensor or field transducer is connected to leads which extend through bore to and beyond the proximal end of body.

The apparatus further includes a set of external field transducers or antennas defining a locating frame of reference. For example, external field transducers may be mounted to a patient-supporting bed. Antennas are linked to a field transmitting and receiving device and a computer which in turn is linked to a display device such as a cathode ray tube. The computer is also provided with conventional input devices such as a keyboard, trackball, mouse and the like. Computer, field transmitting and receiving device and transducers are arranged to cooperate with the probe field transducer to determine the dispositions of the field transducer on the probe, and hence determine the disposition of the distal end of the probe in the locating frame of reference of the external field transducers or antennas . These elements of the apparatus can be as described in the aforementioned '091 or '199 patents. Other devices for detecting disposition of probes equipped with position sensors by transmission of non-ionizing fields are known in the art. As is known in the art, electromagnetic or magnetic fields can be transmitted between an antenna or field transducer mounted in an external frame of reference and a field transducer on a probe, and the disposition of the probe can be calculated from the characteristics of the fields detected by the transducer on the probe. Thus, the external field transducers or antennas and the position sensor or probe field transducer on the probe cooperatively define a plurality of transmitter-receiver pairs. Each such pair includes one transmitter and one receiver as elements of the pair. One element of each such pair is disposed on the probe and the other element of each such pair is disposed at a known disposition in the external frame of reference. Typically, at least one element of each transmitter-receiver pair is disposed at a different position or orientation than the corresponding element of the other pairs. By detecting the characteristics of field transmission between elements of the various pairs, the system can deduce information concerning the disposition of the probe in the external frame of reference. The disposition information can include the position of the probe, the orientation of the probe or both. Although the external field transducers are illustrated as mounted to a rigid structure such as a patient bed, so that the external field transducers remain in fixed position relative to one another, this is not essential. As described in commonly assigned PCT Publication WO 97/29685, the external field transducers may be movable relative to one another. The computer system can determine the positions of the external field transducers by measuring the properties of fields transmitted between these transducers, or between the external field transducers and calibration transducers mounted to the individual external field transducers.

The apparatus further includes a reference field transducer mounted in a protective housing effective to protect field transducer from physical damage when the field transducer is deployed at a position on the outside of a patient's body. Thus, the housing and field transducer can be mounted by any conventional expedient such as adhesive tape, bandages, sutures or the like at a selected point on the exterior of a patient. Optionally, housing may be provided with features such as flat pads or wings suture holes or other physical features which further facilitate attachment to the exterior surface of the body. Reference field transducer has essentially the same structure as probe field transducer discussed above. Leads connect the reference field transducer to the field transmitting and receiving device. The field transmitter and receiver and computer actuate external field transducers and reference field transducer to transmit and receive fields in the same manner as discussed above in connection with probe field transducer. Thus, the system determines the disposition of the reference field transducer in the locating frame of reference defined by external field transducers.

According to one embodiment of the invention, when in use, a patient P is imaged using any conventional imaging modality such as computerized tomographic x-ray ("CAT" or "CT") imaging, magnetic resonance imaging or any other imaging method which is capable of depicting the internal organs of the body and, particularly, the respiratory system and surrounding tissues. The image is acquired while the patient is at a selected respiratory state. The selected respiratory state is a stage of the patient's normal respiratory cycle. Preferably, the image is acquired while the patient is at the so-called "minimum inhalation" stage. This stage is the stage during normal breathing where the patient has exhaled the normal, tidal volume of air. The patient may be instructed to hold his or her breath at the selected state during image acquisition. The image may be a conventional diagnostic image acquired without regard to any special considerations for the therapeutic procedure and indeed acquired before the need for the therapeutic procedure is known.

The image includes at least a portion of the patient's thorax and includes certain features of the patient's anatomy which are readily identifiable in the image with a good degree of precision. These include features of the skeletal system such as the scapula portions of the spine and the sternal notch . In the conventional manner, the image is provided as computer data defining properties of structures of various locations within the body as, for example, x-ray absorption of individual volume elements or "voxels" in a CAT image or MRI data defining magnetic resonance properties such as proton density, T₁ or T₂ for individual voxels.

After the image has been acquired, the patient is placed into position in the locating frame of reference defined by external field transducers as, for example, by placing the patient in supine position on the supporting table. Reference field transducer is then engaged successively with several of the aforementioned readily defined points on the patient's body as, for example, with each of the scapula, with the sternal notch or with readily identified points on the spine. This is done while the patient remains in position on the table. The table may be provided with apertures or grooves in its surface to allow insertion of the reference field transducer into engagement with features of the patient's back. While the reference field transducer is in engagement with each defined point in the patient's anatomy, the external field transducers field transmitting and receiving apparatus and computer are actuated to determine the location of the reference field transducer in the locating frame of reference defined by the external field transducers. Thus, the locations of the various defined points in the locating frame of reference are provided to the computer.

An operator can also input the locations of the same defined points in the frame of reference of the image. For example, computer can be actuated to display depictions of the image which include the various identifiable points in the anatomy and the operator can manually adjust a cursor on the image as, for example, by adjusting a knob, trackball or mouse incorporated in input devices. When the cursor is aligned with an identifiable point in all dimensions, the operator enters a further signal indicating to the computer that the coordinates of the cursor in the image frame of reference correspond to the coordinates of the particular point in the anatomy. Once the coordinates of the identifiable points in the anatomy have been provided to the computer in the image frame of reference and in the locating frame of reference, the computer can derive a mathematical transformation between the locating frame of reference and the image frame of reference. Techniques for acquiring locations of points in the anatomy and deriving transformations between an image frame of reference and a locating frame of reference are well known and are described in the aforementioned patents and publications. In a variant of such techniques, also described in these patents and publications, fiducial markers incorporating field transducers are mounted on the patient before the imaging procedure, so that the fiducial markers are visible in the image. The positions of the fiducial markers in the locating frame of reference are acquired by actuating the field transducers on the fiducial markers in conjunction with the external field transducers, in the same manner as described above. In other variants, the system acquires a succession of positions in the locating frame of reference while a reference field transducer is moved over a well-defined contour in the patient's anatomy. The computer system uses automatic pattern-matching techniques to find a feature having a contour including a set of locations in the image frame of reference which can be mapped to the set of locations in the locating frame of reference by a rigid-body transformation. Again, various techniques for finding matching points in both frames of reference, and for deriving a transformation between the locating and imaging frames of reference, are well known in the art.

The reference field transducer is then mounted on a point on the outside of the patient's chest which moves during respiration. For example, the reference field transducer can be taped or sutured in place over one of the patient's ribs. While the patient remains in position on a table, the patient breathes normally and hence reference field transducer moves cyclically in a motion corresponding to the various phases of the respiratory cycle. Thus, the location of reference field transducer varies with time. The computer, in cooperation with the field transmitting and receiving unit and external field transducers continually monitors the position of reference field transducer. The computer tracks the position of the reference field transducer over time and generates a plot of the reference field transducer position in a selected direction versus time. In practice, the plot consists of a series of numbers denoting the location of the reference transducer along the selected axis at various times. The axis selected for tracking may be a vertical axis (towards and away from the table) and hence towards and away from the patient's back; a horizontal axis transverse to the longitudinal (head-to-toe) axis of the patient or an axis at an arbitrary angle between the vertical and the horizontal.

The computer selects successive extreme positions in the plot. For example, where the location represented by the plot is location in a vertical axis corresponding to movement towards and away from the patient's back, the computer may be actuated to select successive minima of such location, *i*.*e*., the points where the reference field transducer is closest to the patient's back. Alternatively, where the location represented by the plot is horizontal location, the computer may be actuated to select minima in the plot corresponding to locations where the reference field transducer is closest the central axis of the patient. These minima can be found by conventional computer-programming techniques for selecting local minimum values in a sequence of numbers. Numerical techniques of this nature are well known in the programming arts and are available in many standard mathematical software packages. The minima represent the minimum inspiration point in the patient's respiratory cycle as discussed above.

The value of the location at successive minima may not be exactly the same. However, for a patient breathing normally, all of the minima will have values close to one another. Thus, the computer calculates a mean value representing the mean location of several successive minima. The system then applies a preselected tolerance or maximum deviation. Whenever the location of reference field transducer deviates from the mean value by less than a predetermined tolerance the system treats the patient as being at the minimum inspiration point of the respiratory cycle. Thus, by monitoring the respiratory cycle, the system establishes a particular respiratory state corresponding to an extreme of the movement of the reference transducer encountered in normal respiration.

In use, the physician advances the distal end of the probe into the respiratory system of the patient in the conventional manner. Typically, the distal end of the probe is advanced through an airway as, for example, through the larynx and trachea into the bronchi. Computer, field transmit and receive unit and external field transducers cooperate with probe field transducer to determine the position of the probe field transducer and hence the position of the probe distal end in the locating frame of reference defined by the external field transducers, and cooperate with reference transducer to determine its position. When the position of the reference transducer is within the predetermined tolerance of the mean minimum inspiration location, the computer captures the location of the probe field transducer and probe distal end in the locating frame of reference defined by the external field transducers. Thus, the computer captures the location of the probe distal end when the patient is at the minimum inspiratory state.

The computer transforms the location of the probe distal end into the frame of reference of the image and prepares a composite display including at least a portion of the image and a representation of the probe superposed on the image. For example, the displayed image on cathode ray tube may include a depiction of the portion of the airway together with a representation of the probe distal end. The image also includes a depiction of the target tissue, in this case a lesion outside of the airway but adjacent thereto. Preferably, the image displayed is a perspective view, so that the physician can readily perceive the spatial relationships between the distal end of the probe and the target tissue. The system may also generate a line or arrow on the displayed image showing the trajectory from the probe distal tip to the target. The physician can use the information shown in the displayed image to bring the probe distal end into engagement with the target. For example, the physician can bring the distal end of the probe body into engagement with the airway adjacent the target and can advance the biopsy needle to its extended position and thus pierce the airway wall and engage the target.

Because the location of the probe distal end is captured only when the patient is in the same respiratory state as used in image acquisition, the acquired position of the probe distal end, transformed into the frame of reference of the image accurately represents the relative position of the probe distal end and the surrounding tissues. The patient continues to breathe while the probe is advanced into the airway. A new probe position is acquired on each respiratory cycle when the patient reaches the minimum inhalation state. Each time a new probe position is acquired, the display shown on CRT screen is revised to conform with the new probe position. Thus, the physician can monitor the progress of the probe distal end towards the target tissue. The physician can accurately align the probe with the target tissue.

The procedure discussed above can be varied in many ways. For example, the computer can be adjusted to find the mean location for the maximum inhalation of the patient's respiratory cycle, and the image may be acquired at a similar maximum inhalation state. In a further variant, the computer can select an arbitrary axis for plotting motion of the reference transducer so that the axis is aligned with the principal direction of motion of the reference field transducer during respiration. For example, the computer can first track the location of the reference field transducer using a horizontal, vertical or other preset axis to find maxima and minima in the location on that axis. The computer can then compute the average time between successive maxima or successive minima. That time corresponds to the period of the respiratory cycle. The computer can then test various pairs of locations, each including one point delayed in time by one full period from another point. The computer can then calculate the distance in three dimensional space between each pair of points. The pair of points which has the largest distance lie along the principal direction of movement of the reference field transducer during the respiratory cycle. The computer can then plot location along this direction versus time.

The image may be acquired when the patient is at an abnormal respiratory state such as a maximum forced exhalation obtained by deliberately forcing exhalation with maximum voluntary effort, or a maximum forced inhalation obtained by deliberately forcing maximum inhalation with a maximum voluntary effort. In this case, the state used during image acquisition will not recur during a normal respiratory cycle. Instead, the patient is instructed to repeat the state while the system monitors the location of reference field transducer. After the patient repeats the state in one or more trials, the system records the location of the reference transducer at this state, or the mean location obtained in several trials. While the physician is advancing the probe into the respiratory system, the patient is instructed periodically to repeat the same state. The system acquires the image when the patient is holding his or her breath at the desired respiratory state. An arbitrary respiratory state such as a state midway between the maximum and minimum inhalation in a normal respiratory cycle can also be used. However, the patient typically will not be able to reproduce such an arbitrary state accurately. According to a further embodiment of the invention, when in use, the system can provide guidance to the patient and the physician to aid in duplicating an arbitrary respiratory state. Thus, if the reference transducer is mounted on the patient before the imaging procedure, and if the reference field transducer is visible in the image, the position of the field transducer relative to the identifiable points on the body, such as the scapular spine or sternum will vary with the respiratory state of the patient. For example, if the reference field transducer is mounted to the ribs, the field transducer will move outwardly, away from the central axis of the body as the patient inhales. When the patient is placed on table, in proximity to the external field transducers, the system can track the location of the reference field transducer in the manner discussed above. The position of the reference field transducer can be transformed into the frame of reference of the image. If the patient is in the same respiratory state as was used in image capture, the position of the reference field transducer in the image frame of reference will overlie the depiction of the reference field transducer in the image. In a further variant, two or more reference transducers may be attached to the patient at locations which move towards or away from one another during respiration. The system can track the distance between the reference transducers as a measure of respiratory state.

The physician can use the information as to the respiratory state provided by the reference transducer or transducers to provide feedback to the patient, as, for example, by instructing the patient to inhale or exhale slightly to better match the respiratory state used during image capture. Alternatively, a mechanical respirator can be controlled automatically to achieve superposition between the position of the reference field transducer as determined by the field transmitting and receiving apparatus and the position depicted in the image. Thus, the respirator may be arranged to provide substantially normal breathing followed by periods of forced breath holding and inflation of the lungs to the extent necessary to match the position captured in the image.

In the procedures discussed above, the probe is advanced through the airway. However, the same advantages can be obtained in procedures where the probe is advanced through the skin or through the intestinal track to other organs affected by motion due to respiration.

As these and other variation and combinations of the features discussed above can be utilized without departing from the present invention, the foregoing description of the preferred embodiment should be taken by way of illustration rather than by way of limitation of the invention defined by the claims.

### INDUSTRIAL APPLICABILITY

The present invention can be applied in medical and veterinary procedures.

## Claims

1. Apparatus for performing a medical procedure on the respiratory cycle of a patient comprising:
(a) means for acquiring an image of the patient in an image frame of reference while the patient is at a selected respiratory state, said selected respiratory state being a stage of the patient's normal respiratory cycle;
(b) a probe adapted to be inserted into the respiratory system of the patient, an intrabody medical tool, and a probe field transducer;
(c) a set of external field transducers adapted to define a locating frame of reference, said transducers being linked to a field transmitting and receiving device and to a computer, wherein the computer, field transmitting and receiving device and external field transducers are arranged to cooperate with the probe field transducer to determine the disposition of the probe in the locating frame of reference; and
(d) a reference field transducer which, when in use, is deployed on the outside of the patient's body at a position which moves during respiration and is connected to the field transmitting and receiving device, wherein the computer, field transmitting and receiving device and external field transducers are arranged to cooperate with the reference field transducer to determine its disposition in the locating frame of reference;
wherein the computer is adapted to:
transform at least one of the image and disposition of the probe in said locating frame of reference so as to place the image and the disposition of the probe in a common frame of reference;
display said image of the patient with a representation of the probe superposed thereon at a location corresponding to the disposition of the probe in the common frame of reference;
detect when the patient is in said selected respiratory state by monitoring the position of the reference field transducer;
establish a position of said reference field transducer corresponding to said selected stage of the respiratory cycle by monitoring the position of the reference field transducer over a plurality of respiratory cycles and finding an extreme position of the reference field transducer which recurs in each cycle.

2. The apparatus of claim 1, wherein said extreme position corresponds to minimum inspiration.

3. The apparatus of claim 2 or claim 3, wherein said computer is adapted to detect when the patient is in said selected stage by determining whether the position of the reference field transducer matches said extreme position within a preselected tolerance.

4. The apparatus of any preceding claim, wherein the probe is adapted to be advanced within an airway.

5. The apparatus of claim 4, wherein the probe is adapted to pass a part of the probe through the wall of the airway to sample or treat tissue at a target located within the tissues surrounding said airway.

6. The apparatus of claim 5, wherein said probe includes an endoscope and a needle and is adapted to advance the endoscope until the endoscope is positioned at the wall of the airway adjacent the target location, and then to advance the needle through the wall of the airway.

7. The apparatus of any preceding claim, wherein said image includes a plurality of identifiable points on the body of the patient which have positions substantially unaffected by the respiratory cycle of the patient.

8. The apparatus of claim 7, wherein said identifiable points include a point on the scapula or on the sternum.

9. The apparatus of any preceding claim, wherein said computer is adapted to transform the disposition of the probe into the image frame of reference.

10. The apparatus of claim 9, when dependent on claim 4, wherein the computer is arranged to display a perspective image of the airway and surrounding tissues so that the position of said probe and the trajectory for moving said probe to engage said target location can be visualized by viewing the displayed image.

## Patentansprüche

1. Vorrichtung zum Ausführen eines medizinischen Vorgangs bei dem Atmungszyklus eines Patienten, welche folgendes umfaßt:
(a) eine Einrichtung zum Erfassen eines Bildes des Patienten in einem Referenzbildrahmen, während der Patient sich in einem ausgewählten Atmungszustand befindet, wobei der ausgewählte Atmungszustand ein Zustand des normalen Atmungszyklus des Patienten ist;
(b) eine Sonde, die dafür eingerichtet ist, in das Atmungssystem des Patienten eingeführt zu werden, ein körperintemes medizinisches Gerät und einen Sondenfeldwandler;
(c) einen Satz externer Feldwandler, die dafür eingerichtet sind, einen Referenzortsrahmen festzulegen, wobei die Wandler mit einer feldaussendenden und empfangenden Einrichtung und mit einem Computer verbunden sind, wobei der Computer, die feldaussendende und empfangende Einrichtung und die externen Feldwandler dafür eingerichtet sind, mit dem Sondenfeldwandler zusammenzuwirken, um die Verlagerung der Sonde in dem Referenzortsrahmen zu bestimmen, und
(d) einen Referenzfeldwandler, der, wenn er in Gebrauch ist, auf der Außenseite des Körpers des Patienten an einer Position aufgebracht wird, die sich während der Atmung bewegt, und der mit der feldaussendenden und empfangenden Einrichtung verbunden ist, wobei der Computer, die feldaussendende und empfangende Einrichtung und die externen Feldwandler dafür eingerichtet sind, mit dem Referenzfeldwandler zusammenzuwirken, um die Verlagerung in dem Referenzortsrahmen zu bestimmen;
wobei der Computer dafür eingerichtet ist:
wenigstens eines von dem Bild und der Verlagerung der Sonde in dem Referenzortrahmen zu übertragen, um das Bild und die Verlagerung der Sonde in einem gemeinsamen Referenzrahmen anzuordnen;
das Bild des Patienten mit einer Darstellung der Sonde, die darauf an einem Ort überlagert ist, welcher der Verlagerung der Sonde entspricht, in dem gemeinsamen Referenzrahmen anzuzeigen; zu bestimmen, wann sich der Patient in dem ausgewählten Atmungszustand befindet, durch Überwachen der Position des Referenzfeldwandlers; und
eine Position des Referenzfeldwandlers hervorzubringen, die dem ausgewählten Zustand des Atmungszyklus entspricht, durch Überwachen der Position des Referenzfeldwandlers über mehrere Atmungszyklen und durch Finden einer Extremposition des Referenzfeldwandlers, die in jedem Zyklus wiederkehrt.

2. Vorrichtung nach Anspruch 1, bei dem die Extremposition der minimalen Einatmung entspricht.

3. Vorrichtung nach Anspruch 2 oder 3, bei welcher der Computer dafür eingerichtet ist, zu ermitteln, wann sich der Patient in dem ausgewählten Zustand befindet, durch Bestimmen, ob die Position des Referenzfeldwandlers zu der Extremposition innerhalb einer vorgegebenen Toleranz paßt.

4. Vorrichtung nach einem der vorhergehenden Ansprüche, bei welcher die Sonde dafür eingerichtet ist, in einem Atemweg vorgeschoben zu werden.

5. Vorrichtung nach Anspruch 4, bei der die Sonde dafür eingerichtet ist, einen Teil der Sonde durch die Wand des Atemwegs durchzuführen, um Gewebe an einem Ziel zu prüfen oder zu behandeln, das sich in dem Gewebe befindet, das den Atemweg umgibt.

6. Vorrichtung nach Anspruch 5, bei der die Sonde ein Endoskop und eine Nadel umfaßt und die dafür eingerichtet ist, das Endoskop vorzuschieben, bis das Endoskop an der Wand des Atemweges an dem Zielort angrenzend angeordnet ist, und anschließend die Nadel durch die Wand des Atemweges vorzuschieben.

7. Vorrichtung nach einem der vorhergehenden Ansprüche, bei der das Bild mehrere identifizierbare Punkte auf dem Körper des Patienten umfaßt, die Positionen aufweisen, die von dem Atmungszyklus des Patienten im wesentlichen unbeeinflußt bleiben.

8. Vorrichtung nach Anspruch 7, bei dem die identifizierbaren Punkte einen Punkt auf dem Schulterblatt oder dem Brustbein umfassen.

9. Vorrichtung nach einem der vorhergehenden Ansprüche, bei dem der Computer dafür eingerichtet ist, die Verlagerung der Sonde in den Referenzbildrahmen zu übertragen.

10. Vorrichtung nach Anspruch 9 mit Rückbezug auf Anspruch 4, bei welcher der Computer dafür eingerichtet ist, ein perspektivisches Bild des Atemweges und des umgebenden Gewebes anzuzeigen, sodaß die Position der Sonde und die Trajektorie zum Bewegen der Sonde, um an dem Zielort anzuliegen, durch Betrachten des angezeigten Bildes visualisiert werden kann.

## Revendications

1. Appareil permettant d'effectuer une procédure médicale concernant le cycle respiratoire d'un patient, comprenant :
(a) un moyen permettant d'acquérir une image du patient dans un cadre de référence d'image pendant que le patient se situe à un état respiratoire sélectionné, lequel état respiratoire sélectionné est un état du cycle respiratoire normal du patient ;
(b) une sonde conçue pour être insérée dans le système respiratoire du patient, un instrument médical intra-corporel et un transducteur de champ de la sonde ;
(c) un ensemble de transducteurs de champ externes conçus pour définir un cadre de localisation de référence, lesquels transducteurs sont reliés à un dispositif d'émission et de réception de champ ainsi qu'à un ordinateur, l'ordinateur, le dispositif d'émission et de réception de champ ainsi que les transducteurs de champ externes étant disposés de manière à coopérer avec le transducteur de champ de la sonde de manière à déterminer la disposition de la sonde dans le cadre de localisation de référence ; et
(d) un transducteur de champ de référence qui, lors de l'utilisation, est déployé sur l'extérieur du corps du patient en une position qui se déplace pendant la respiration, et qui est connecté au dispositif d'émission et de réception de champ, l'ordinateur, le dispositif d'émission et de réception de champ ainsi que les transducteurs de champ externes étant conçus pour coopérer avec le transducteur de champ de référence afin de déterminer sa disposition dans le cadre de localisation de référence ;
appareil dans lequel l'ordinateur est conçu pour :
- transformer l'image et/ou la disposition de la sonde dans ledit cadre de localisation de référence de manière à placer l'image et la disposition de la sonde dans un cadre de référence commun ;
- afficher ladite image du patient avec une représentation de la sonde superposée à celle-ci en un endroit correspondant à la disposition de la sonde dans le cadre de référence commun ;
- détecter si le patient se situe dans ledit état respiratoire sélectionné en contrôlant la position du transducteur de champ de référence ;
- établir une position dudit transducteur de champ de référence correspondant au dit état sélectionné du cycle respiratoire en contrôlant la position du transducteur de champ de référence au cours de plusieurs cycles respiratoires ; et
- trouver une position extrême du transducteur de champ de référence qui revient à chaque cycle.

2. Appareil selon la revendication 1, dans lequel ladite position extrême correspond à l'inspiration minimale.

3. Appareil selon la revendication 1 ou la revendication 2, dans lequel ledit ordinateur est conçu pour détecter le moment où le patient se situe dans ledit état sélectionné en déterminant si oui ou non la position du transducteur de champ de référence correspond à ladite position extrême selon une tolérance prédéterminée.

4. Appareil selon l'une quelconque des revendications précédentes, dans lequel la sonde est conçue pour être avancée dans une voie respiratoire.

5. Appareil selon la revendication 4, dans lequel la sonde est conçue pour qu'une partie de la sonde passe à travers la paroi de la voie respiratoire de manière à échantillonner ou traiter des tissus au niveau d'une cible située dans les tissus entourant lesdites voies respiratoires.

6. Appareil selon la revendication 5, dans lequel ladite sonde comprend un endoscope et une aiguille, et est conçue de manière à faire avancer l'endoscope jusqu'à ce que celui-ci soit positionné au niveau de la paroi de la voie respiratoire à proximité de l'emplacement cible, puis à faire avancer l'aiguille à travers la paroi de la voie respiratoire.

7. Appareil selon l'une quelconque des revendications précédentes, dans lequel ladite image comprend plusieurs points identifiables sur le corps du patient, dont les positions ne sont essentiellement pas affectées par le cycle respiratoire du patient.

8. Appareil selon la revendication 7, dans lequel les points identifiables comprennent un point sur l'omoplate ou sur le sternum.

9. Appareil selon l'une quelconque des revendications précédentes, dans lequel ledit ordinateur est conçu pour transformer la disposition de la sonde dans le cadre de référence de l'image.

10. Appareil selon la revendication 9, lorsqu'elle dépend de la revendication 4, dans lequel l'ordinateur est conçu de manière à afficher une image en perspective de la voie respiratoire et des tissus environnants de sorte que la position de ladite sonde et la trajectoire de déplacement de ladite sonde pour qu'elle entre en contact avec ledit emplacement cible, puissent être visualisées en regardant l'image affichée.
